# EUROPEAN PATENT SPECIFICATION

(11) **EP 0 322 384 B1**
(45) Date of publication and mention of the grant of the patent: **13.03.1996**
(21) Application number: 88850370.3
(22) Date of filing: 27.10.1988
(51) Int. Cl.: C07H 19/04, C07H 19/06, A61K 31/70

(54) **Nucleosides for use in therapy**
Nucleoside zur Verwendung für die Therapie
Nucléosides pour utilisation en thérapie

(30) Priority: 03.11.1987 SE 8704298
(43) Date of publication of application: 28.06.1989
(73) Proprietor: Medivir Aktiebolag, S-141 44 Huddinge (SE)
(72) Inventor: Johansson, Karl Nils Gunnar, S-15023 Enhörna (SE); Lindborg, Björn Gunnar, S-125 42 Älvsjö (SE); Norinder, Ulf, S-151 54 Södertälje (SE); Stening, Göran Bertil, S-151 50 Södertälje (SE)
(74) Representative: Dost, Wolfgang, Dr.rer.nat., Dipl.-Chem.

(56) References cited:
- EP-A- 0 217 580
- EP-A- 0 254 268
- DE-A- 3 045 375
- US-A- 3 282 921
- US-A- 3 687 931
- J Med Chem 30 (1987) 440-4
- J Med Chem 32 (1989) 1743-9
- Antimicrob. Agents Chemother 38 (1994) 1590-1603
- Patent Abstract of Japan. vol. 9, no. 192, (C-296), abstract of JP 60-61591, published 9 April 1985 (TAIHOU YAKUHIN KOGYO K.K.)
- Patent Abstract of Japan, Vol. 8, no. 118, (C-226), abstract of JP 59-29699, published 16 February 1984 (FUNAI YAKUHIN KOGYO K.K.)
- Chemical Abstracts, vol. 102, no. 23, 10 June 1985, (Columbus, Ohio, US), page 629, abstract 204245t
- JOURNAL F. PRAKT. CHEMIE, Vol. 315, 1973, no. 5, pages 895-900
- JOURNAL OF MEDICINAL CHEMISTRY, vol. 30, 1987, pages 1270-1278
- BIOCHEMICAL AND BIOPHYSICAL RESEARCH COMMUNICATIONS, vol. 46, no. 4, 1972, pages 1734-1741
- JOURNAL OF THE AMERICAN CHEMICAL SOCIETY, vol. 95, no. 24, 28 November 1973, pages 8146-8156
- CHEMISCHE BERICHTE, vol. 109, 1976, pages 1395-1406, R. Mengel et al
- CANCER BIOCHEM. BIOPHYS. 1976, vol. 1, pages 203-209

## Description

The present invention relates to the use of known chemical compounds and pharmaceutically acceptable salts thereof in the manufacture of a medicament in therapy for therapheutic and prophylactic control and treatment of the Acquired Immuno Deficiency Syndrome (AIDS), infections by Human Immunodeficiency Virus, hepatitis B virus infections and other retrovirus infections and method for such control and treatment in mammals and man.

### Background of the invention

In the late seventies a new disease was reported, which subsequently was referred to as Acquired Immuno Deficiency Syndrome (AIDS). It is now generally accepted that a retrovirus referred to as HIV (Human Immunodeficiency Virus), formerly known as Human T-cell Lymphotropic Virus (HTLV-III) or Lymphadenopathy Associated Virus (LAV) plays an essential role in the etiology of AIDS. Different types of HIV have been found, such as HIV-1 and HIV-2 and more are likely to be isolated.

AIDS is characterized by a profound immunodeficiency due to low numbers of a subset of lymphocyte-T-helper cells, which are one target for HIV infection. The profound immunodeficiency in AIDS patients makes these patients highly susceptible to a variety of opportunistic infections of bacterial, fungal, protozoal or viral etiology. The etiological agents among viral opportunistic infections are often found in the herpes virus group, i.e. Herpes simplex virus (HSV), Varicella Zoster virus (VZV), Epstein-Barr virus (EBV) and, especially, cytomegalovirus (CMV). Other retroviruses affecting humans are HTLV-I and II and examples of retroviruses affecting animals are feline leukemia virus and equine infectious anaemia virus. Human diseases such as multiple sclerosis, psoriasis and Kawasaki disease have also been reported to be associated with retrovirus infections.

Hepatitis B virus infections cause severe disease such as acute hepatitis, chronic hepatitis, fulminant hepatitis in a considerable number of persons. It is estimated that there are 200 million patients with chronic hepatitis B infection in the world. A considerable number of the chronic cases progress to liver cirrosis and liver tumours. In some cases the hepatitis infections also take a rapid and severe course as in fulminant B hepatitis with about 90% mortality. At present there is no known effective treatment against hepatitis B infections. The replication of hepatitis B virus is similar to that of retroviruses and it contains the same essential viral reverse transcriptase activity.

### General outline of the invention

A great number of nucleoside analogues exhibit several antimetabolic activities. They do so by substituting for or competing with the naturally occuring nucleosides. Recently some nucleoside analogues have been described, which inhibit in cell culture the multiplication of human immunodeficiency virus (HIV, also called HTLV-III, LAV), the causative agent of AIDS and AIDS-related complex (ARC).

We have now found that activities for inhibition of HIV multiplication are exhibited by nucleoside analogues, in which the nucleoside bases are both natural and modified and the sugar moieties have the 2′-deoxyribofuranosyl structure with the 3'-substituent being an electronegative, non hydroxylic functionality.

### Prior Art

Nucleoside analogues which have an inhibitory effect on the multiplication of HIV are described in EP-A 0-199 451, EP-A 0 196 185, DE-A 3 608 606 and EP-A 0-217 580, referring to 3′-azidonucleosides, such as azidothymidine, of the general formula 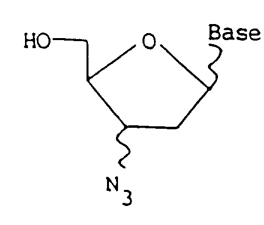 Lin et al. (J. Med. Chem. 30, 440-444 (1987)) disclose two 3'-azido analogues of thymidine exhibiting an activity against retroviruses, in particular the socalled HTLV-III/LAV/AAV ("AIDS"-virus). The compounds are 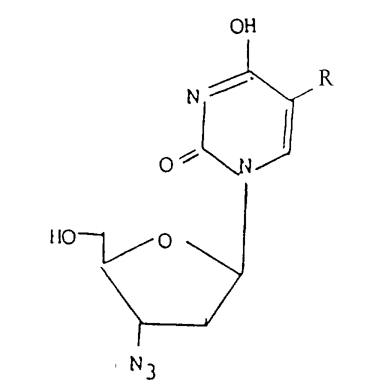 wherein R it either Br or I.

In EP-A 0-206 497 2′,3′-dideoxynucleosides are described to have good activity against different human viruses such as HTLV and hepatitis B virus. Said compounds are of the general formula

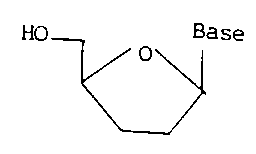

The following compounds are known:

1. The compounds of the formula 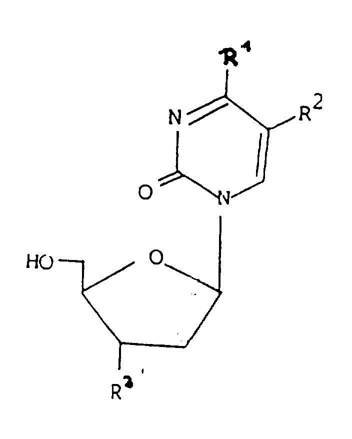 wherein
   R¹ = OH or NH₂, R = H or CH₃ (with the provisos that R is H, if R¹ is NH₂ and that R = CH₃, if R¹ = OH),
   and R³ = F are described by P. Herdewijn et al, J. Med. Chem. 30 (1987) 1270-1278 to have an anti-HIV activity. Such anti-AIDS agents are also known from EP 0 277 151 (published on January 14, 1988).
2. The compound of the formula 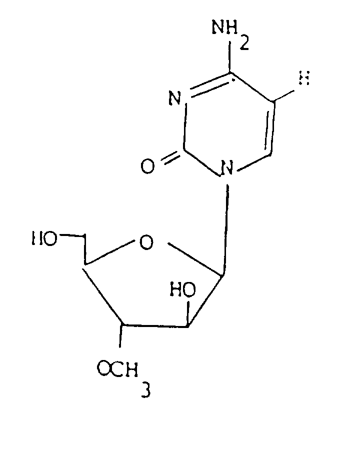 is described by E. Darzynkiewicz, J. T. Kuzmierek, D. Shugar, Biochem. Biophys. Res.Commun. 1972 46(4) 1734-41; and by M. Remin, D. Shugar, J. Am. Chem. Soc. 95 (1973)8146. Reference to an anti-HIV activity has not been made in any of them.
3. The compound of the formula 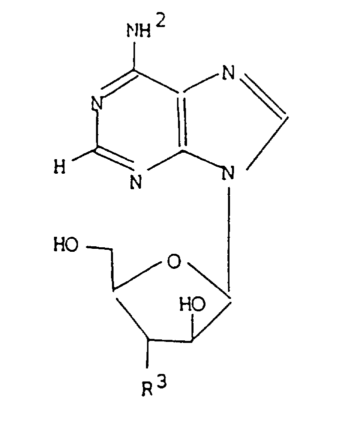 wherein
   R³ = F is described by K. Miyai, R. K. Robins, R. L. Tolman, J. Med. Chem. 15 (1972) 1092;
   R³ = Cl, Br, I by R. Mengel, H. Wiedner, Chem. Ber 109 (1976) 1395;
   R³ = OCH₃ by E. Darzynkiewicz, Z. Kazimierczuk, D. Shugar, Cancer, Biochem., Biophys. 1 (1976) 203.
   Again, none of these references mentions an anti-HIV activity of the disclosed nucleosides.
4. Compounds of the general formula, 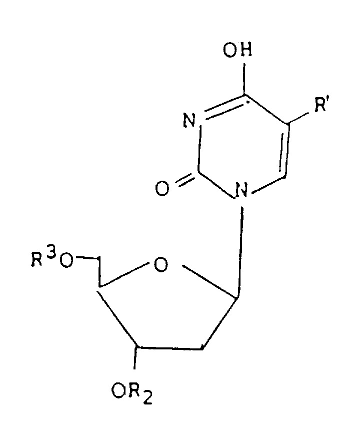 wherein R¹ is F or CF₃, any of R and R³ is H and the other is e.g. an alkyl group having 1 to 10 carbon atoms, are known as antiviral and antitumor agents. No activity against HIV is mentioned.
5. From E. Darzynkiewicz and D. shugar, Acta Bioch Pol. 21 (1974) 305-322 the following compounds are known 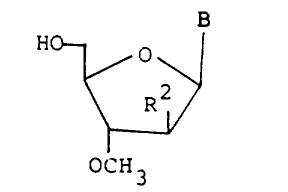 wherein B is uracil or cytosine and R is OH or OCH₃. Nothing is stated as to medical use.
6. EP 254 268, which was published on January 27, 1988, and EP 277 150) refer to compounds of the formula 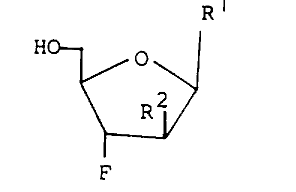 wherein R¹ is a N-1-glycosidically bonded radical derived from thymine, uracil, cytosine or a 5-substituted derivative thereof such as the C₂H₅- or Br derivative; and R is H or OH, as an anti-AIDS drug.
7. EP 88903972.3, published on April 6, 1988 as WO88/08001, refers to compounds of the formula 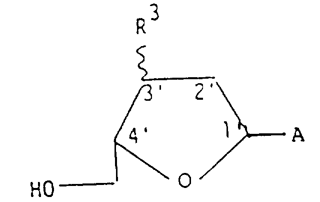 wherein A is 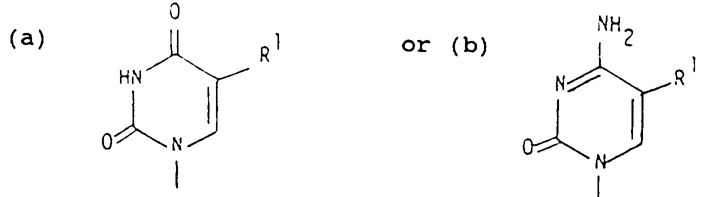 wherein R¹ is H; alkyl containing 1-3 carbon atoms, including cyclopropyl; -CH=CH₂; -CH=CH-CH₃; -CH₂-CH=CH₂ ; 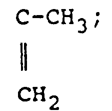 -C≡CH, and R³ is F, CN, C≡CH, OCH₃, CH₂OH, said compounds exhibiting properties which make them suitable for the prophylaxis and/or treatment of AIDS.
8. Other compounds known from the art which have some anti-HIV activity are those having a double bond between carbon atoms 2 and 3 in the sugarring and a glycosidically bonded cytosine (EP 0261 595) or thymidine residue (EP 0 273 277) attached thereto.
9. Finally, from DeClercq et al., J. Carbohydrates, Nucleosides, Nucleotides 5, 187 (1978) compounds of the formula 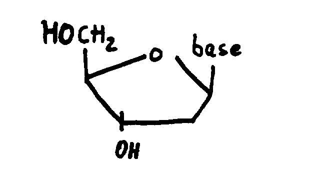 have been described to be useful as anti-retroviral drugs.

### Disclosure of the invention

It has been found according to the present invention that a compound of the formula 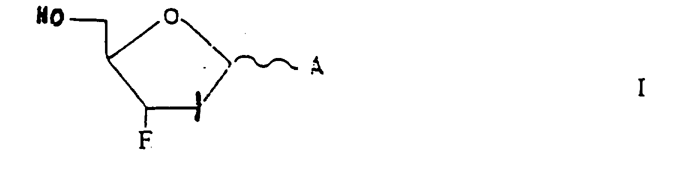 wherein A is 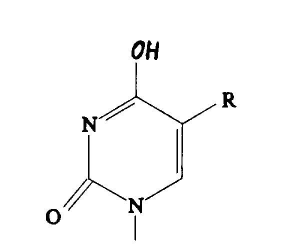 and the radical R is defined as follows:
R : F, CF₃, Cl, CH₂OCH₃, CH₂SCH₃, CH₂SH
and pharmaceutically acceptable salts thereof, inhibit the multiplication of human immunodeficiency virus (HIV). The compounds of the formula I are useful as therapeutic and/or prophylactic agents in the control and treatment of HIV virus infections in mammals and man.

In a more general aspect, the compounds of the formula I are useful as therapeutic and/or prophylactic agents in the control and treatment of infections caused by retroviruses and hepatitis B virus in mammals and man.

All retroviruses, including HIV, require the enzyme reverse transcriptase in their natural cycle of replication.

Hepatitis B virus (HBV) is a DNA virus with a unique circular double-stranded DNA genome which is partly single-stranded. It contains a specific DNA polymerase required for viral replication. This DNA polymerase also acts as a reverse transcriptase during the replication of HBV DNA via an RNA intermedidate.

The compounds of the formula I inhibit the activity of reverse transcriptase of retroviruses including HIV as well as the activity of DNA polymerase-reverse transcriptase of hepatitis B virus.

The present invention has the following aspect:
use of a compound of the formula I in the manufacture of a medicament for therapeutic and/or prophylactic treatment of infections caused by a retrovirus, including HIV, or by hepatitis B virus.

It is a preferred aspect of the invention to combat HIV virus infections in man.

The invention comprises α-anomers as well as β-anomers of the compounds of formula I. In α-anomers the -CH₂ OH residue at the 5'position and A are in trans-configuration and in β-anomers in cis-configuration.

Preferred compounds of the formula I are those wherein

A is 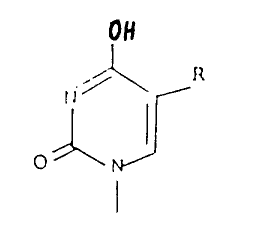 wherein R = F or CF₃.

Esters and ethers can be formed by the nucleosides of the invention. Examples of esters are phosphate esters, carboxylic esters, carbonate esters or sulphonic esters. The acid part of the esters may have alkyl, aryl or arylalkyl chains, where the aryl functionalities are optionally substituted for example by alkoxy, amino, nitrile, alkyl or sulphonamido groups or by one or more halogen atoms. Examples of other types of derivatives of the nucleosides are alkyl or arylalkyl derivatives of the 5' hydroxyl group. The arylalkyl ether derivatives may be for example benzyl or triphenyl methyl and the aryl moiety may be optionally substituted. Furthermore, it is understood that the examples of the pharmaceutically acceptable salts cited below also apply to the various esters or derivatives of the nucleosides of the invention.

In a compound of the formula I CH₂OH as an ether residue can be defined as CH₂OR⁴, wherein R⁴ is C₁₋₆ alkyl, arylalkyl optionally substituted with one or more alkoxy, amino, nitrile or sulphonamido groups or one or more halogen atoms.

CH₂OH as an ester residue can be derived from a carboxylic acid R⁹COOH, a carbonic acid R¹⁰˙O˙COOH, a sulphonic acid R¹⁰SO₂˙OH or a phosphoric acid, wherein R⁹ is H, C₁₋₁₇ alkyl, preferably C₁₋₆ alkyl, arylalkyl or aryl and R¹⁰ is C₁₋₁₇ alkyl, preferably C₁₋₆ alkyl, arylalkyl or aryl, and said aryl and arylalkyl groups optionally can be substituted with one or more alkyl, alkoxy, amino, nitrile, sulphonamido groups or one or more halogen atoms.
Examples of pharmaceutically acceptable salts of the compounds of formula I include base salts, e.g. derived from an appropriate base, such as alkali metal (e.g. sodium), alkaline earth metal (e.g. magnesium) salts, ammonium and NX₄ ⁺ (wherein X is C₁₋₄ alkyl). Physiologically acceptable salts of a hydrogen atom or an amino group include salts of organic carboxylic acids such as acetic, lactic, gluconic, citric, tartaric, maleic, malic, pantothenic, isethionic, succinic, oxalic, lactobionic and succinic acids; organic sulfonic acids such as methanesulfonic, ethanesulfonic, benzenesulfonic, p-chlorobenzenesulphonic and p-toluenesulfonic acids and inorganic acids such as hydrochloric, hydroiodic, sulfuric, phosphoric and sulfamic acids. Physiologically acceptable salts of a compound of an hydroxy group include the anion of said compound in combination with a suitable cation such as Na⁺, NH₄ ⁺, and NX₄ ⁺ (wherein X is a C₁₋₄ alkyl group).

In clinical practice the nucleosides of the formula I will normally be administered orally, by injection or by infusion in the form of a pharmaceutical preparation comprising the active ingredient in the form of the original compound or optionally in the form of a pharmaceutically acceptable salt thereof, in association with a pharmaceutically acceptable carrier which may be a solid, semi-solid or liquid diluent or an ingestible capsule. The compound may also be used without carrier material. As examples of pharmaceutical preparations may be mentioned tablets, dragées, capsules, granulates, suspensions, elixirs, syrups, solutions, liposomes etc. Usually the active substance will comprise between 0.05 and 20% for preparations intended for injection and between 10 and 90% for preparations intended for oral administration.

In the treatment of patients suffering from retrovirus, especially HIV, or hepatitis B virus infections, it will be preferred to administer the compounds by any suitable route including the oral, parenteral, rectal, nasal, topical and vaginal route. The parenteral route includes subcutaneous, intramuscular, intravenous and sublingual administration. The topical route includes buccal and sublingual administration. The dosage at which the active ingredients are administered may vary within a wide range and will depend on various factors such as the severity of the infection, the age of patient etc., and may have to be individually adjusted. As a possible range for the amount of the compounds of the invention or a physiologically acceptable salt thereof to be administered per day may be mentioned from about 10 mg to about 10 000 mg, preferentially 100-500 mg for intravenous administration and preferentially 100-3000 mg for oral administration.

Compounds of the formula I cooperate synergistically or additively with a wide range of other therapeutic agents, thereby enhancing the therapeutic potential of both agents without adding the toxic effects, thus increasing the therapeutic ratio.

Therefore, a compound of formula I or a pharmaceutically acceptable derivative thereof can be used in combination therapy, wherein the two active agents are present in a ratio resulting in an optimal therapeutic ratio. This can be provided either by a synergistic effect against the viral infection and/or by a decrease in toxicity while maintaining a therapeutic effect which is additive or synergistic. A combination therapy can also be of use to obtain effects on different types of cells infected with HIV and requiring different drugs for effect.

The optimal therapeutic ratio is observed when the two agents are present in a ratio of 500:1 to 1:500, preferably 100:1 to 1:100, particularly 20:1 to 1:20 and especially 10:1 to 1:10.

Said combinations may conveniently be administered together, for example, in a unitary pharmaceutical formulation, or separately, for example as a combination of tablets and injections administered at the same time or different times, in order to achieve the required therapeutic effect.

The compounds of the formula I are potentiated by interferons, reverse transcriptase inhibitors, such as foscarnet, AZT, glucuronidation inhibitors, renal excretion inhibitors, HIV protease inhibitors, immunomodulators, interferon inducers and growth factors.

Particularly preferred types of interferon are α, β and γ and interferon inducers such as "Ampligen" (Hem Research).

Probenecid is particularly useful in combination with compounds of the formula I, as it possesses both renal excretion inhibiting activity and glucuronidation blocking activity. Examples of other compounds useful in this aspect include acetaminophen, aspirin, lorazepam, cimetidine, ranitidine. zomepirac, clofibrate, indomethacin, ketoprofen, naproxen and other compounds which compete for glucuronidation or otherwise undergo significant glucuronidation.

Other combinations suitable for use according to the present invention include those wherein the second agent is, for example, interleukin II, suramin, foscarnet or an ester thereof, HPA 23, inhibitors of HIV protease such as pepstatin, steroids, medications such as levamisol or thymosin to increase lymphocyte numbers and/or function as appropriate, or GM-CSF and other factors regulating cell functions.

### Methods of preparation

The compounds useful for the manufacture of a medicament according to the invention may be prepared by one of the following general methods:
A. Condensing a glycoside as comprised in formule I where the hydroxyl groups may be optionally protected, to the N-1 position of a pyrimidine derivative according to known methods described in the literature. Such methods are described for example in "Basic Principles in Nucleic Acid Chemistry", Vol. 1 (Academic Press, 1974, Ed. P.O.P.Ts'o), in "Nucleoside Analogues, Chemistry, Biology and Medical Applications" (Pharma Press, 1979, Eds. R I Walker, E. De Clercq and F. Eckstein).

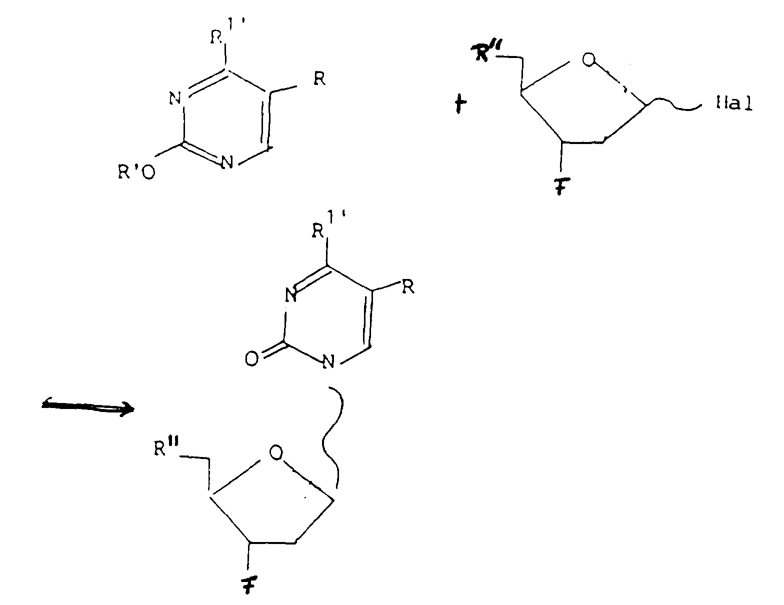

   Examples of suitable derivatives of the reacting species are those wherein R′ is C₂H₅ or (CH₃)₃Si; R^{1'} is OC₂H₅, (CH₃)₃SiO, or N(COCH₃)Si(CH₃)₃; dimethyl, tert-butylsilyl); R" is OR⁴ wherein R⁴ is silyl (e.g. dimethyl, tert-butylsilyl), except that now it must not be H; R is are as defined above.
   After condensation the products may be hydrolyzed or converted by conventional methods, known to those skilled in the art, into compounds of the formula I. The glycosides are known or may be prepared by suitable adaptions of known methods. The synthesis of a 2,3-dideoxy-3-fluoro-erythro-pentofuranoside for example, has been described by G.W.J. Fleet and J.C. Son in Tetrahedron Letters 40 (1987) pp 3615-3618.
B. The fluorine in the 3' position of the glycon moiety may be introduced by substitution of a hydroxyl group or a suitably derivatized hydroxyl group 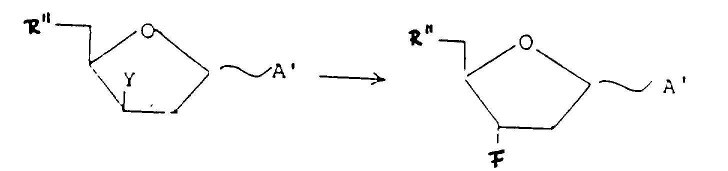
   wherein A' is 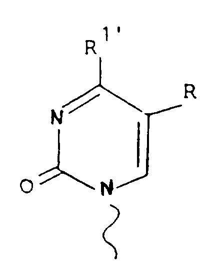 Y is OH or a functionality that will be leaving in the substitution reaction such as for example CF₃SO₃; and R¹', R , and R" are as defined above.

The epoxide opening may be performed by conventional methods described in the literature and known to those skilled in the art.

The epoxide compound may be prepared from condensation of the nucleobase or nucleobase analogue with 1-O-acetyl-D-ribofuranose or -xylofuranose with protecting groups on the oxygen atoms, followed by transformation of the ribofuranosyl or the xylofuranosyl ring into a 2',3'-anhydro-lyxofuranosyl moiety by known methods.

The following examples will further illustrate the invention.

### Example I

### Preparation of 1-(2',3'-anhydro-5'-O-trityl-β-D-lyxofuranosyl)thymine (VSB-020)

The title compound was prepared by a 5-step sequence reaction. Thymine (12,6 g) was treated with hexamethyldisilazane and 1-O-acetyl-2,3,5-tri-O-benzoyl-β-D-ribofuranose (50 g) was added. The 1-(2',3',5'-tri-O-benzoyl-β-D-ribofuranosyl)thymine obtained was treated with sodium methoxide and gave 1-(β-D-ribofuranosyl)thymine (23 g). Then said substance was treated with tritylchloride and methylsulfonylchloride and 1-(2',3'--di-O-methyleulfonyl-5'-O-trityl-β-D-ribofuranoeyl)thymine (39 g) was obtained. Said substance was treated with sodium hydroxide and gave the title compound 1-(2'-3'-anhydro-5'-O-trityl-β-D-lyxofuranosyl)thymine (VSB-020) 19.2 g. Yield 43%. ¹H-NMR (DMSO-d₆) δ: 1.66 (s, 3H, 5-CH₃), 3.24-3.34 (m, 2H, 5'-H), 4.08 (s, 2H, 2'-H and 3'-H), 4.25 (t, 1H, 4'-H), 6.13 (s, 1H, 1'-H), 7.30-7.45 (m, 16H, trityl H and 6-H), 14.46 (s, 1H, N-H).

### Example II

### Preparation of 1-(3'-deoxy-3'-ethynyl-β-arabinofuranosyl)-thymine (VSB-029)

a) A suspension of an ethylene diamine complex of ethynyl lithium (275 mg, 3 mmol) in dry DMSO (5 ml) was stirred under nitrogen for 1 h at 20°C. A solution of 1-(2',3'-anhydro-5'-O-trityl-β--D-lyxofuranosyl)thymine (VSB-020; 482 mg) in dimethylsulfoxide (DMSO; 2 ml) was added with a syringe to the reaction suspension. The reaction mixture was kept at 20°C for 48 h. The reaction mixture was then poured into a saturated ammonium chloride solution (100 ml) and extraced with ethyl acetate (2 x 100 ml). The organic phase was washed with a saturated ammonium chloride solution (100 ml), brine (100 ml) and water (100 ml) and dried over disodium sulfate. The solvent was evaporated and the residue purified on a silica gel column using toluene:ethyl acetate (7:4 v/v) as eluent. The appropriate fractions were collected and evaporation of the solvent gave 300 mg of 1-(3'-deoxy-3'-ethynyl-5'-O-trityl-β-D-arabinofuranosyl)thymine (VSB-028). Yield 59%. R_{f} 0.45 (silica, ethyl acetate).
b) A solution of 1-(3'-deoxy-3'-ethynyl-5'-O-trityl-β-D-arabinofuranosyl)thymine (VSB-028; 280 mg) in acetic acid (8 ml) and water (2 ml) was heated at 80°C for 15 min. Then, the solvent was evaporated and the residue purified on a silica gel column, using first chloroform as the eluent followed by chloroform:ethanol (9:1 v/v). The combined fractions were evaporated and the residue was triturated with hexane:ethanol (9:1 v/v) to give 110 mg of the title compound (VSB-029). Yield 75%. M.p. 198-199°C.

¹H-NMR (DMSO-d₆) δ: 1.75 (s, 3H, 5-CH₃), 2.89 (t, 1H, 3'-H), 3.18 (d, 1H, -C≡C-H), 3.55-3.79 (m, 3H, 4'-H and 5′-H), 4.41 (q (t on D₂O shake), 1H, 2′-H), 5.29 (t, 1H, 5′-OH), 5.97 (d, 1H, 2′-OH), 6.03 (d, J=6.22 Hz, 1H, 1′-H), 7.65 (s, 1H, 6-H), 11.25 (s, 1H, N-H).

### Example III

### Preparation of 1-(3'-cyano-3'deoxy-β-D-arabinofuranosyl)-thymine (VSB-026)

a) To a solution of VSB-020, 1-(2',3'-anhydro-5'-O-trityl-β-D-lyxofuranosyl)thymine (480 mg) in dry dimethylsulfoxide (DM S0) (10 ml) was added sodium cyanide (245 mg). The reaction mixture was heated at +45°C for 15 h, whereafter a saturated ammonium chloride solution (50 ml) was added to the reaction mixture. The mixture was extracted with ethyl acetate (3 x 50 ml) and the combined organic layers were washed with a saturated ammonium chloride solution (50 ml); brine (50 ml) and H₂O (50 ml), dried over disodium sulfate and purified on a silica gel column using toluene:ethyl acetate (7:4 v/v) as eluent to give 160 mg of 1-(3'-cyano-3'-deoxy-5'-O-trityl-β-D-arabinofuranosyl)-thymine (VSB-025). Yield 31%. R_{f} O.47 (silica, ethylacetate).
b) A solution of 1-(3'-cyano-3'-deoxy-5'-O-trityl-β-D-arabinofuranosyl)-thymine (VSB-025; 200 mg) in acetic acid (8 ml) and water (2 ml) was heated at 80°C for 15 min. Then, the solvent was evaporated and the residue purified on a silica gel column, using first chloroform as the eluent followed by chloroform:- ethanol (9:1 v/v). The combined fractions were evaporated and the residue was triturated with hexane:ethanol (9:1 v/v) and gave 49 mg of the title compound VSB-026. Yield 60%. M.p. 219-221°C dec.

¹H-NMR (DMSO-d₆) δ: 1.75 (s, 3H, 5-CH₃), 3.23 (t, 1H, 3'-H), 3.50-3.80 (m, 2H, 5′-H), 4.09 (d, 1H, 4′-H), 4.72-4.86 (m (t on D₂O shake), 1H, 2′-H), 5.38 (t, 1H, 2′-OH), 6.10 (d, J = 6.22 Hz, 1H, 1′-H), 6.34 (d, 1H, 5′-OH), 7.54 (s, 1H, 6-H), 11.30 (s, 1H, N-H).

### Example IV

### Preparation of 1-(2′-3′-anhydro-β-D-lyxofuranosyl)-thymine (VSB-024)

1.5 g of 1-(2′,3′-anhydro-5′-O-trityl-β-D-lyxofuranosyl)thymine (VSB-020) was treated with 80% acetic acid (10 ml) at +95°C for 15 min. The solvent was then removed by evaporation and the residue was purified on a silica gelcolumn using ethyl acetate as the eluent and 790 mg of the title compound VSB-024 was obtained. Yield 93%. M.p. 144-146°C.
¹H-NMR (DMSO-d₆) δ: 1.80 (s, 3H, 5-CH₃), 3.60 (s, 1H, 5'-CH), 3.61 (d, 2H, 5′-H), 3.96-4.06 (m, 3H, 2'-H, 3′-H and 4′-H), 6.03 (s, 1H, 1'-H), 7.45 (s, 1H, 6-H), 11.20 (broad s, 1H, N-H).

### Example V

### Preparation of 1-(3'-deoxy-3'-fIuoro-β-D-arabinofuranosylthymine (VSB-027)

A mixture of 1'-(2',3'-anhydro-5'-O-trityl-β-D-lyxofuranosyl)thymine (VSB-024; 367 mg), potassium hydrogenfluoride (586 mg) in dry ethane-1,2-diol (10 ml) was heated at +200°C for 30 min. After cooling, the reaction mixture was directly applied to a silica gel column. The column was eluted with chloroform:ethanol (9:1 v/v), and 210 mg of the title compound (VSB-027) was obtained. Further purification by trituration with hexane : ethyl acetate gave 190 mg of VSB-027. Yield 48%. M.p, 162-164°C (EtOH).
¹H-NMR (DMSO-d₆) δ: 1.76 (s, 3H, 5-CH₃), 3.65 (t (d on D₂O shake), 2H, 5'-H), 4.04 (d, J 3'F, 4' = 23 Hz, 1H, 4'-H), 4.34 (d, J 3'F, 2' = 16Hz, 1H, 2'-H), 4.97 (d, J3'F, 3' = 53 Hz, 1H, 3'-H), 5.24 (t, 1H, 2'-OH), 6.00 (d, J4Hz, 1H, 1'H), 7,43 (s, 1H, 6-H), 11.35 (s, 1H, N-H).

## Claims

1. Use of a compound of the formula 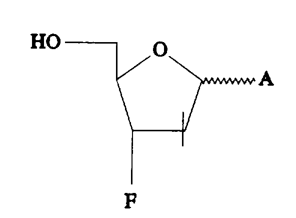 wherein A is 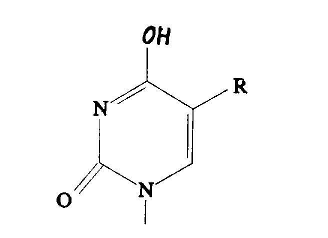 and the radical R is defined as follows:
R: F, CF₃, Cl, CH₂OCH₃, CH₂SCH₃, CH₂SH
and pharmaceutically acceptable salts thereof, for the manufacture of a medicament for the therapeutic and/or prophylactic treatment of infections in mammals and man caused by a retrovirus, including HIV, or HBV.

2. The use according to claim 1, wherein R = F or CF₃.

## Patentansprüche

1. Verwendung einer Verbindung der Formel 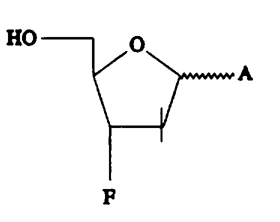 wobei der Rest A 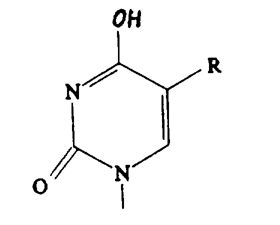 ist und der Rest R wie nachstehend definiert ist:
R: F, CF₃, Cl, CH₂OCH₃, CH₂SCH₃, CH₂SH
und pharmazeutisch verträglicher Salze davon zur Herstellung eines Medikaments zur therapeutischen und/oder prophylaktischen Behandlung von Infektionen in Säugern und im Menschen, die verursacht werden durch ein Retrovirus, einschließlich HIV oder HBV.

2. Verwendung nach Anspruch 1, wobei der Rest R=F oder CF₃.

## Revendications

1. Utilisation d'un composé ayant la formule 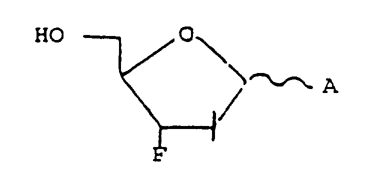 dans lequel A est 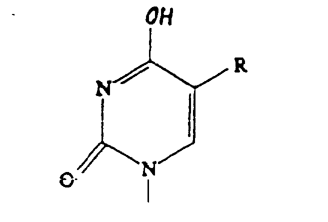 et le radical R est défini comme suit :
R : F, CF₃, Cl, CH₂OCH₃, CH₂SCH₃, CH₂SH
et de sels de celui-ci pouvant être acceptés de manière pharmaceutique, destinés à la fabrication d'un médicament pour le traitement thérapeutique et/ou prophylactique d'infections causées par un rétrovirus, incluant le HIV, ou par le HBV chez des mammifères et chez l'homme.

2. Utilisation selon la revendication 1, dans laquelle R = F ou CF₃.
